(19) Europäisches Patentamt
European Patent Office
Office européen des brevets

(11) Numéro de publication: **0 223 674**
**A1**

# (12) DEMANDE DE BREVET EUROPEEN

(21) Numéro de dépôt: **86402355.1**

(22) Date de dépôt: **20.10.86**

(51) Int. Cl.[4]: **C 07 D 265/36**
C 07 D 265/34,
C 07 D 413/06, A 61 K 31/535

(30) Priorité: **21.10.85 FR 8515595**

(43) Date de publication de la demande:
**27.05.87 Bulletin 87/22**

(84) Etats contractants désignés:
**AT BE CH DE ES FR GB GR IT LI LU NL SE**

(71) Demandeur: **LABORATOIRES NEGMA**
**ZAC de Buc 584 rue Fourny**
**F-78530 Buc (FR)**

(72) Inventeur: **Lesieur, Isabelle**
**20, rue de Verdun**
**F-59147 Gondecourt (FR)**

**Lespagnol, Charles**
**115, avenue Pasteur**
**F-59130 Lambersart (FR)**

**Moussavi, Ziaddine**
**1, rue Baden Powell**
**F-59130 Lambersart (FR)**

**Fruchart, Jean-Claude**
**23, Domaine de la Hollande**
**F-59320 Ennetieres en Weppes (FR)**

**Sauzieres, Jacques**
**29, avenue de Paris**
**F-78000 Versailles (FR)**

**Monfilliette, Nicole**
**24/14, rue des Vergers**
**F-59560 Villeneuve D'ASC (FR)**

(74) Mandataire: **Gutmann, Ernest et al**
**S.C. Ernest Gutmann - Yves Plasseraud 67, boulevard Haussmann**
**F-75008 Paris (FR)**

(54) **Acyl-7 benzoxazinones et leurs dérivés, procédé pour les obtenir et compositions pharmaceutiques les contenant.**

(57) Produits de formule générale:

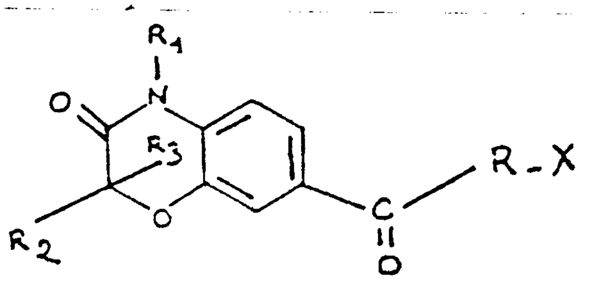

dans laquelle
- R est un groupe hydrocarboné formé d'éléments cycliques ou aliphatiques, ou des deux à la fois, comprenant de 1 à 15 atomes de carbone, ou l'un des susdits groupes hétérocycliques,
- X est de l'hydrogène ou un groupe carboxyle, halogène, hydroxyle, nitro, aminé, lui-même substitué ou non par un ou deux groupes alcoyle comprenant de 1 à 4 atomes de carbone ou un groupe aminé hétérocylique, alcoxyle comprenant de 1 à 4 atomes de carbone, X pouvant encore définir une liaison chimique de cyclisation formée entre R et l'atome de carbone en position 6 du cycle benzénique de la partie benzoxazinone de la molécule, ainsi que, s'il y a lieu, les sels ou esters correspondants et les isomères optiques lorsque $R_2$ et $R_3$ sont différents, et quand X est l'hydrogène ou le brome, d'une part, et R est $CH_2$, d'autre part, $R_1$, $R_2$ et $R_3$ ne peuvent être simultanément tous de l'hydrogène.

Les compositions pharmaceutiques, administrables notamment par voie orale, contenant les susdits composés en association avec un véhicule pharmaceutique acceptable, sont utiles comme médicament.

EP 0 223 674 A1

**Description**

ACYL-7 BENZOXAZINONES ET LEURS DERIVES, PROCEDE POUR LES OBTENIR ET COMPOSITIONS PHARMACEUTIQUES LES CONTENANT.

L'invention concerne des acyl-7 benzoxazinones (ou 7-acyl (2H)-1,4-benzoxazine-3 (4H) ones) et des dérivés de celles-ci, et un procédé pour les obtenir.

Les produits de l'invention sont dépourvus de toxicité et présentent un grand éventail de propriétés pharmacologiques permettant leur application dans différents domaines thérapeutiques.

Ils présentent notamment des propriétés antiathéroscléreuses importantes ainsi que des propriétés analgésiques, anti-inflammatoires, anti-microbiennes et antifongiques, cardiovasculaires et des propriétés sur le système nerveux central.

L'invention concerne donc également des compositions pharmaceutiques contenant ces produits en association avec un véhicule pharmaceutiquement acceptable.

Les produits selon l'invention sont caractérisés par la formule générale :

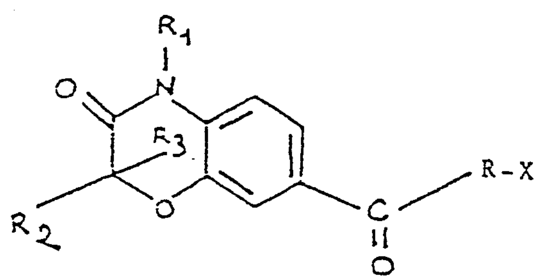

dans laquelle :

$R_1$ est soit de l'hydrogène, soit un groupe hydrocarboné comprenant de 1 à 6 atomes de carbone, de préférence alkyle, alkaryle ou aralkyle, le cas échéant porteurs de substituants, notamment du type de ceux envisagés ciaprès à propos du groupe X, soit un group hétérocyclique, notamment thiényle, furyle, pyridinyle ou pyridyle,

- $R_2$ et $R_3$ sont chacun, et ce de façon indépendante l'un de l'autre, de l'hydrogène ou un groupe alcoyle comprenant de 1 à 6 atomes de carbone,
- R est un groupe hydrocarboné formé d'éléments cycliques ou aliphatiques, ou des deux à la fois, comprenant de 1 à 15 atomes de carbone, ou l'un des susdits groupes hétérocyliques,
- X est de l'hydrogène ou un groupe carboxyle, halogène, hydroxyle, nitro, aminé, lui-même substitué ou non par un ou deux groupes alcoyle comprenant de 1 à 4 atomes de carbone ou un groupe aminé hétérocyclique, alcoxyle comprenant de 1 à 4 atomes de carbone, X pouvant encore définir une liaison chimique de cyclisation formée entre R et l'atome de carbone en position 6 du cycle benzénique de la partie benzoxazinone de la molécule, ainsi que, s'il y a lieu, les sels ou esters correspondants, et les isomères optiques lorsque $R_2$ et $R_3$ sont différents.

De préférence, $R_1$, $R_2$ et $R_3$ sont, indépendamment les uns des autres, des hydrogènes ou des groupes méthyle. Dans une première classe préférée de composés, ci-après désignée pour la commodité du langage sous l'expression "composés du groupe I", R est un groupe aryle notamment phényle, arylalkyle ou hétérocyclique, alkaryle notamment benzyle, alkyle y compris cyclopropyle, comprenant de 1 à 6 atomes de carbone ou l'un des susdits groupes hétérocycliques, X étant de l'hydrogène ou un halogène, notamment le chlore ou le brome, un hydroxyle ou un groupe aminé, notamment la pipérazine, substitué ou non, ou encore l'un des susdits groupes hétérocycliques.

Une seconde classe préférée de composés selon l'invention est formée de "cétones alpha-bêta éthyléniques" dans lesquelles le groupe R est une chaîne aliphatique dont le premier carbone, en alpha du groupe carbonyle est porteur d'un groupe de ramification éthyllénique, notamment méthylène. De préférence le groupe CO-R est alors un groupe méthylène-2 butyryle. Ces composés seront quelquefois ci-après désignés par l'expression "composés du groupe II".

Dans une troisième classe de composés préférés selon l'invention - formée par des "Chalcones" ou

"composés du groupe III", le groupe R est un groupe -C=C-W-X, dans laquelle W est un groupe alkyle, arylalkyle ou aryle, notamment -C=C-$C_6H_5$-X, X étant de l'hydrogène, un halogène, un groupe amino ou hydroxyle, méthoxyle ou nitro.

Dans une quatrième classe de composés, du type indanones benzoxazinoniques (ci-après dénommée "composés du groupe IV"), R est une chaîne aliphatique comprenant 2 ou 3 atomes de carbone, éventuellement ramifiée par un groupe $R_4$ (de préférence alcoyle) comprenant de 1 à 4 atomes de carbone, et formant une liaison de cyclisation de cette chaîne avec l'atome de carbone en position 6 sur le cycle benzénique du groupe benzoxazinone.

La structure générale des "indanones benzoxazinoniques" est alors la suivante :

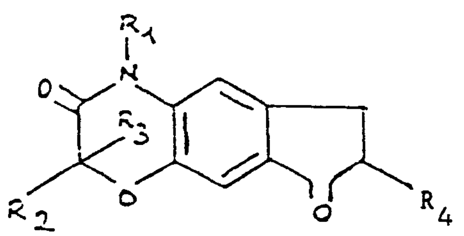

Les composés selon l'invention sont accessibles à partir des acyl-5 amino-2 phénols décrits dans le brevet 73.23282 déposé par l'Institut National de la Santé et de la Recherche Médicale ou encore dans Eur. J. Med. Chem. 1974, 9, 491, et présentant la formule :

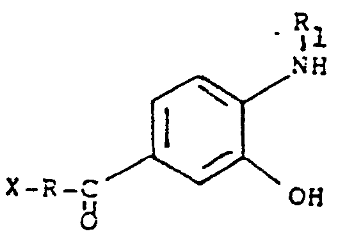

dans laquelle $R_1$ a la signification sus-indiquée, R et X ont les significations correspondant à celles qui ont été indiquées plus haut.

Les acyl-5-amino-2- phénols, dans lesquels $R_1$ est de l'hydrogène, ont été obtenus par hydrolyse des acyl-6 benzoxazolinones correspondant et dans les conditions décrites dans le brevet. Pour produire les acyl-5-amino-2 phénols dans lesquels $R_1$ est par exemple un groupe méthyle, on a mis en oeuvre le mode opératoire suivant :

- chauffer les dérivés acyl-6 méthyl-3 benzoxazolinoniques correspondants dans une solution aqueuse de soude à 20% pendant une heure à 80°C (jusqu'à dissolution dans le milieu). Filtrer et acidifier à chaud par une solution d'acide chlorhydrique concentré, puis alcaliniser par une solution de carbonate de sodium jusqu'à pH 8 ; essorer le précipité, et laver par de l'eau jusqu'à neutralité du filtrat et recristalliser dans un solvant convenable.

Un procédé préféré de l'invention pour obtenir les "composés du groupe I" comprend une réaction de condensation entre un dérivé de l'aminphénol correspondant de formule :

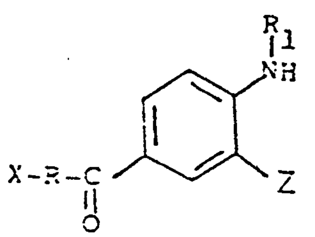

dans laquelle $R_1$, et X ont les significations sus-indiquées et Z est de l'hydrogène (la réaction étant alors de préférence conduite en milieu alcalin) un métal alcalin, notamment le sodium, et un ester alphahalogéné de formule :

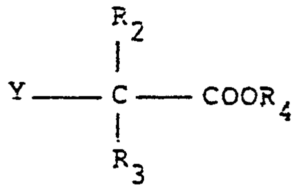

dans laquelle $R_2$ et $R_3$ ont les significations sus-indiquées, Y est un halogène, notamment le brome, et $R_4$ un groupe alkyle, notamment méthyle ou éthyle, alkaryle, aralkyle ou aryle.

On remarquera que ce procédé est également directement applicable à la fabrication des composés des autres groupes.

Un procédé préféré pour obtenir les composés du groupe II est obtenu en soumettant des composés du groupe I dans lesquels R-X forment un groupe alkyle ou arylalkyle, par exemple n-propyle, à une réaction en présence de formol et d'amine en milieu d'acide acétique ou en présence d'un réactif tel que le N'N'N'N'-tétraméthyl diamino-méthane dans le même milieu.

Pour former les "chalcones" ou "composés du troisième groupe", un procédé préféré consiste, partant d'un "composé du premier groupe", à le traiter au sein d'un solvant organique par l'aldéhyde X-W-CHO, dans lequel X et W ont les significations sus-indiquées, en particulier l'aldéhyde-benzoïque.

Pour former les composés de la quatrième classe préférée sus-indiquée, celle des indanones benzoxzinoniques, on réalise une cyclisation de la cétone alpha, bêta éthylénique (ou propylénique) correspondante en milieu acide concentré (notamment dans une solution d'acide sulfurique concentrée).

Il apparaîtra immédiatement à l'homme de métier que d'autres variantes de ce procédé de fabrication (ou de passage d'un composé conforme à l'invention à un autre composé conforme à l'invention) peuvent être envisagées, sans que ces variantes ne cessent de constituer des équivalents des variantes préférées qui ont été définies dans ce qui précède.

Dans une cinquième classe préférée de composés selon l'invention R est un groupe hydrocarboné aliphatique contenant au moins 3 atomes de carbone et de préférence au moins 4.

Des caractéristiques supplémentaires de l'invention apparaîtront encore au cours de la description qui suit de modes de mise en oeuvre des différentes variantes du procédé selon l'invention qui ont été décrites, et des caractéristiques à la fois chimiques et biologiques des produits obtenus à titre d'exemples.

## ACYL-7 BENZOXAZINONES

## STRUCTURE GENERALE

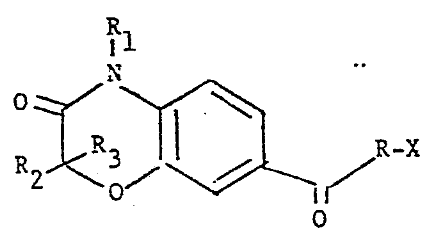

EXEMPLE 1

Préparation des "composés du groupe I".

Le mode opératoire général suivant a été utilisé :

Les matières premières de ces dérivés sont des amino-2 acyl-5 phénols.

Dans une fiole de 100 cm3, dissoudre 0,03 mole de l'aminophénol dans 60 cm3 de DMSO. Porter sous agitation magnétique, ajouter 0,03 mole d'éthylate de sodium de façon à former le phénate. Ajouter goutte à goutte 0,03 mole d'un ester α halogéné

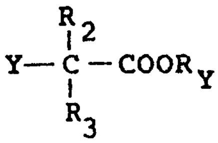

dans lequel Y, $R_2$, $R_3$ et $R_4$ ont les significations susindiquées. Adapter une garde à chlorure de calcium et poursuivre l'agitation pendant trois heures à température ambiante. Verser le milieu réactionnel dans 500 cm3 d'eau glacée. Essorer le précipité formé, laver à l'eau jusqu'a neutralité du filtrat. Sécher et recristalliser le précipité dans un solvant convenable. La mise en oeuvre de ce procédé a permis d'obtenir les produits suivants :

1/ <u>Benzoyl-7 benzoxazinone (MZ 114</u>)

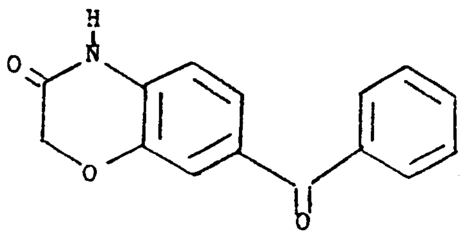

P.M. = 253,25 pour $C_{15}H_{11}NO_3$
P.F. = 208-210°C
Solvant de recristallisation : acétone
Rendement : 75-80%

2/ <u>Benzoyl-7 méthyl-2 benzoxazinone (MZ 115</u>)

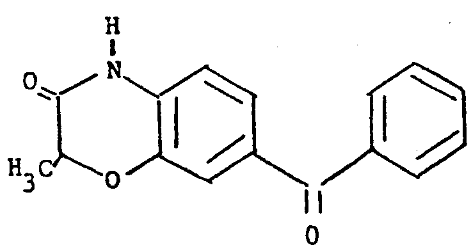

P.M. = 267,27 pour $C_{16}H_{13}NO_3$
P.F. = 158-160°C
Solvant de recristallisation : alcool à 95°
Rendement : 75-80%

3/ Benzoyl-7 diméthyl-2,2 benzoxazinone (MZ 116)

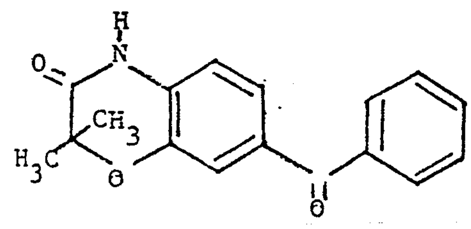

P.M. = 281,29 pour $C_{17}H_{15}NO_3$
P.F. = 212°C
Solvant de recristallisation : acétone ou acétonitrile
Rendement : 70%

4/ Benzoyl-7 méthyl-4 benzoxazinone (MZ 117)

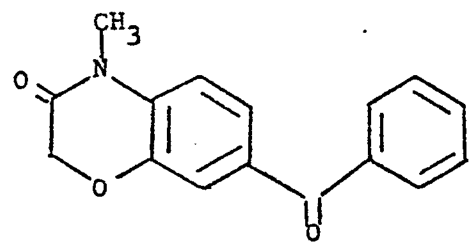

P.M. = 267,27 pour $C_{16}H_{13}NO_3$
P.F. = 118°C
Solvant de recristallisation : méthanol
Rendement : 80%

5/ Benzoyl-7 diméthyl-2,4 benzoxazinone (MZ 118)

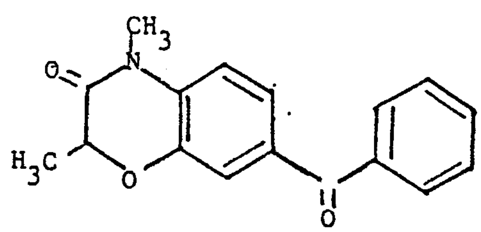

P.M. = 281,30 pour $C_{17}H_{15}NO_3$
P.F. = 128°C
Solvant de recristallisation : méthanol
Rendement : 75%

6/ Benzoyl-7 triméthyl-2,2,4 benzoxazinone (MZ 119)

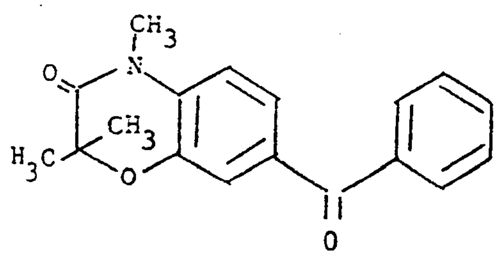

P.M. = 295,32 pour $C_{18}H_{17}NO_3$
P.F. = 122-124°C
Solvant de recristallisation : méthanol
Rendement : 75%

7/ (Chloro-4 benzoyl)-7 benzoxazinone (MZ 120)

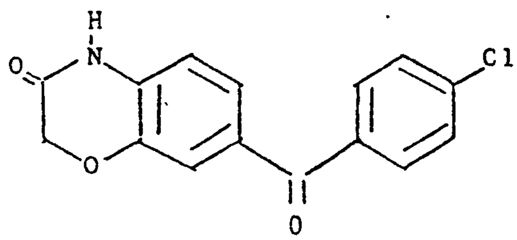

P.M. = 287,74 pour $C_{15}H_{10}NO_3CL$
P.F. = 242°C
Solvant de recristallisation : alcool à 95° ou acétate d'éthyle
Rendement : 50%

8/ (Chloro-4 benzoyl)-7 méthyl-2 benzoxazinone (MZ 121)

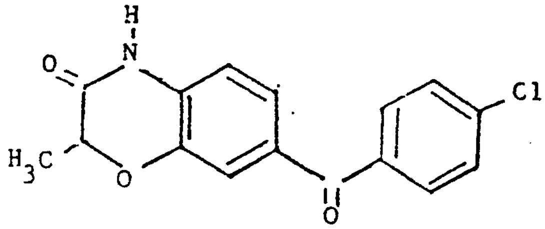

P.M. = 301,76 pour $C_{16}H_{12}NO_3Cl$
P.F. = 185°C
Solvant de recristallisation : acétone ou alcool absolu ou alcool à 95°
Rendement : 60-65%

9/ <u>(Chloro-4 benzoyl)-7 dimethyl-2,2 benzoxazinone</u>
(MZ 122)

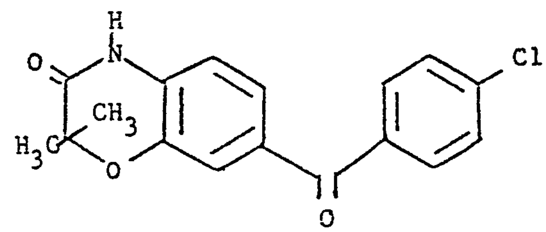

P.M. = 315,79 pour $C_{17}H_{14}NO_3Cl$
P.F. = 185-187°C
Solvant de recristallisation : alcool à 95°
Rendement : 60-65%

10/ <u>(Chloro-4 benzoyl)-7 méthyl-4 benzoxazinone</u>
(MZ 123)

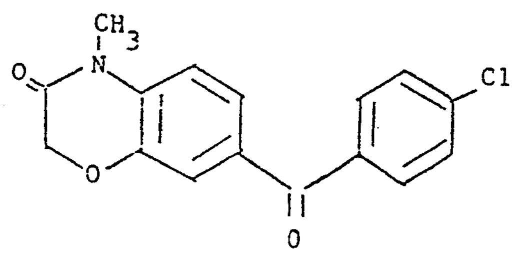

P.M. = 301,76 pour $C_{16}H_{12}NO_3Cl$
P.F. = 190-192°C
Solvant de recristallisation : benzène
Rendement : 70%

11/ <u>(Chloro-4 benzoyl)-7 diméthyl-2,4 benzoxazinone</u>
(MZ 124)

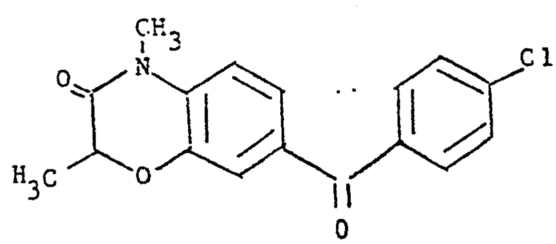

P.M. = 315,79 pour $C_{17}H_{14}NO_3Cl$
P.F. = 113-115°C
Solvant de recristallisation : alcool absolu
Rendement : 70%

12/ Chloro-4 benzoyl)-7 triméthyl-2,2,4, benzoxazinone (MZ 125)

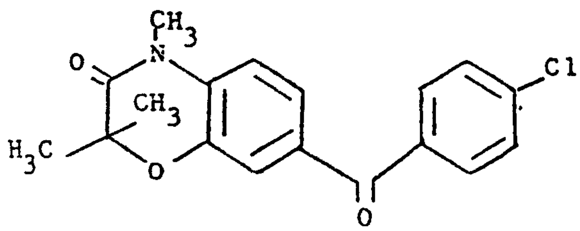

P.M. = 329,81 pour $C_{18}H_{16}NO_3Cl$
P.F. = 130-132°C
Solvant de recristallisation : alcool absolu ou alcool à 95°
Rendement : 60%

13/ Acétyl-7 diméthyl-2,4 benzoxazinone (MZ 132)

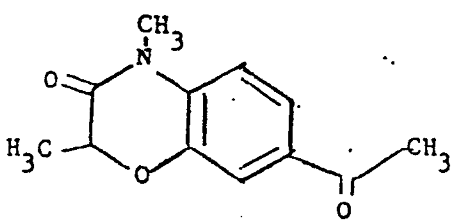

P.M. = 219,23 pour $C_{12}H_{13}NO_3$
P.F. = 132-134°C
Solvant de recristallisation : méthanol
Rendement : 75%

14/ Acétyl-7 triméthyl-2,2,4 benzoxazinone (MZ 136)

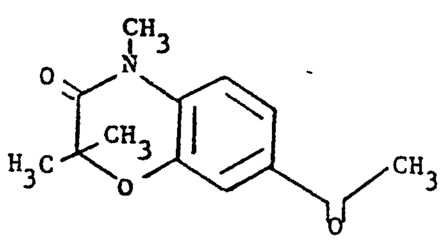

P.M. = 233,25 pour $C_{13}H_{15}NO_3$
P.F. = 100-102°C
Solvant de recristallisation : 1 - hexane
2- alcool absolu
Rendement : 75%

15/ Acétyl-7 méthyl-4 benzoxazinone (MZ 126)

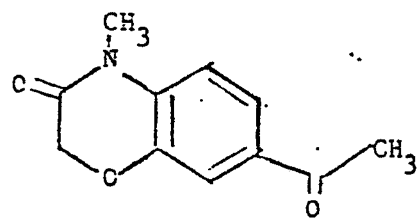

P.M. = 205,20 pour $C_{11}H_{11}NO_3$
P.F. = 150°C
Solvant de recristallisation : alcool absolu ou méthanol

Dérivés d'acétyl 7 benzoxazinone

Mode opératoire

Dissoudre dans 60 cm³ d'éthanol absolu 1 équivalent d'acétyl-7 benzoxazinone, 1,3 équivalent d'amine diversement substituée et un équivalent de trioxyméthylène. Ajouter au mélange 1 goutte d'acide chlorhydrique concentré.
Laisser à reflux du solvant pendant 40 heures. Ajouter aprés 1 H 30 et 19 H de reflux 0,5 équivalent de trioxyméthylène.
Refroidir, essorer et laver le précipité par de l'acétone-recristallisée.

16/ Chlorhydrate de [morpholino-3 propionyl]7 triméthyl 2,2,4 benzoxazinone (NM7)

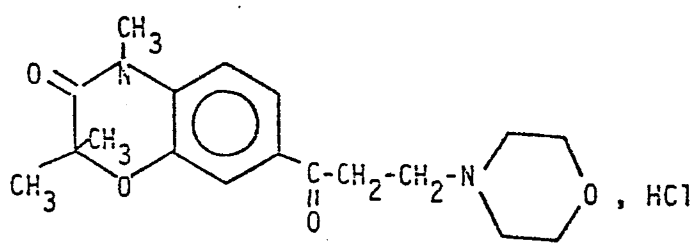

P.M. = 368,83 pour $C_{18}H_{25}ClN_2O_4$
P.F. = 202-204°C
Solvant de recristallisation : éthanol absolu
Rendement : 48%

17/ [(Metatrifluorométhylphényl-4 piperazino)3] propionyl-7 triméthyl 2,2,4 benzoxazinone, chlorhydrate (NM 8)

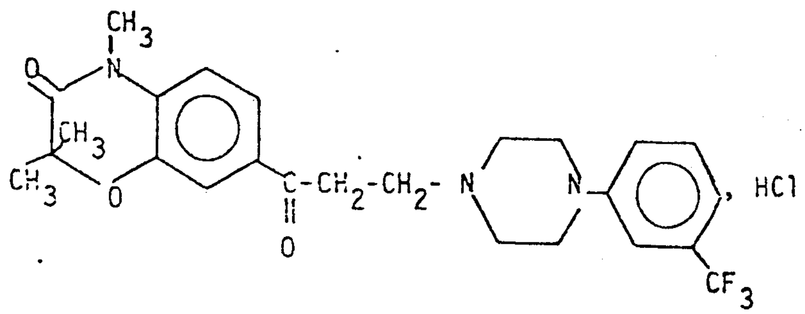

P.M. = 511,97 pour $C_{25}H_{29}CLF_3N_3O_3$
P.F. = 198°C
Solvant de recristallisation = éthanol absolu
Rendement : 55%

18/ Butyryl-7 diméthyl-2,2 benzoxazinone (MZ 95)

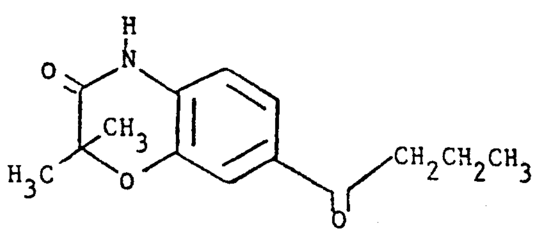

P.M. = 247,28 pour $C_{14}H_{17}NO_3$
P.F. = 137-139°C
Solvant de recristallisation : méthanol
Rendement : 70%

19/ Butyryl-7 méthyl-4 benzoxazinone (MZ 81)

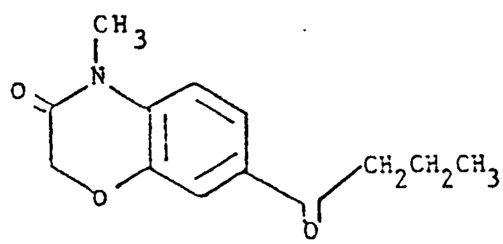

P.M. = 233,26 pour $C_{13}H_{15}NO_3$
P.F. = 104-105°C
Solvant de recristallisation : cyclohexane ou alcool absolu
Rendement : 85%

20/ Butyryl-7 diméthyl-2,4 benzoxazinone (MZ 85)

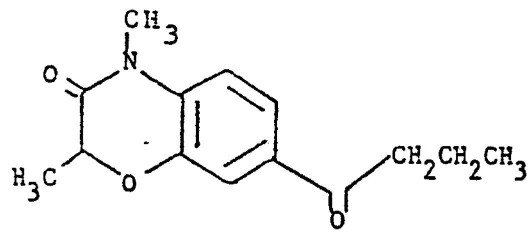

P.M. = 247,28 pour $C_{14}H_{17}NO_3$
P.F. = 84°C
Solvant de recristallisation : hexane ou cyclohexane
Rendement : 75%

21/ Butyryl-7 triméthyl-2,2,4 benzoxazinone (MZ 86)

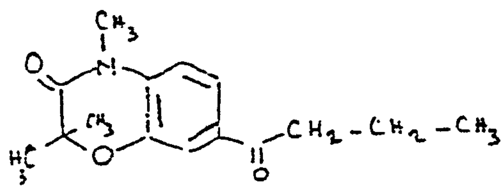

P.M. = 261,30 pour $C_{15}H_{19}NO_3$
P.F. = 80°C
Solvant de recristallisation : hexane ou alcool absolu
Rendement : 75%

22/ Lauryl-7 diméthyl 2,4 benzoxazinone (NM4)

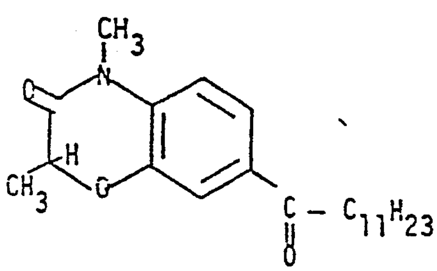

P.M. = 359,48 pour $C_{22}H_{33}NO_3$
P.F. = 68-69°C
Solvant de recristallisation : alcool à 95°C
Rendement : 55%

23/ Lauryl-7 triméthyl 2,2,4 benzoxazinone (NM3)

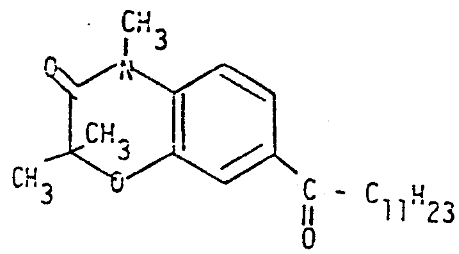

P.M. = 373,50 pour $C_{23}H_{35}NO_3$
P.F. = 57-58°C
Solvant de recristallisation : méthanol
Rendemant : 55%

24/ Valeroyl-7 diméthyl 2,4 benzoxazinone (NM 17)

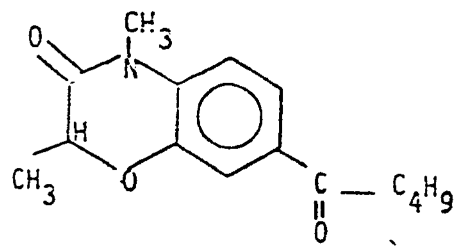

P.M. = 261,30 pour $C_{15}H_{19}NO_3$
P.F. = 76°
Solvant de recristallisation : hexane
Rendement : 70%

25/ Butyryl-7 ß cyanoéthyl-4 diméthyl 2,2 benzoxazinone (NM 14)

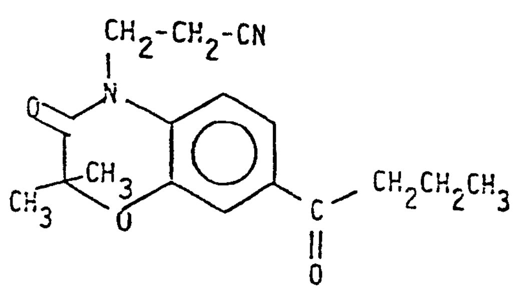

Mode opératoire :

Dans un erlen rodé, dissoudre sous agitation 0,01 mole de butyryl-7 diméthyl 2,2 benzoxazinone (MZ 95) dans 100 cm³ d'éther. Ajouter goutte à goutte 0,02 mole d'acrylonitrile sous une vive agitation puis 0,4 cm³ de Triton B. Laisser 36 heures au reflux, sous agitation. Filtrer la solution éthérée et évaporer l'excès d'éther. Essorer le précipité obtenu, le rincer à l'éther, sécher et recristalliser.
P.M. = 300,36 pour $C_{17}H_{20}N_2O_3$

P.F. = 90-91°C
Solvant de recristallisation : Hexane
Rendement : 86%

26/ Butyryl 7 β carboxyéthyl 4 diméthyl 2,2 benzoxazinone
(NM 11)

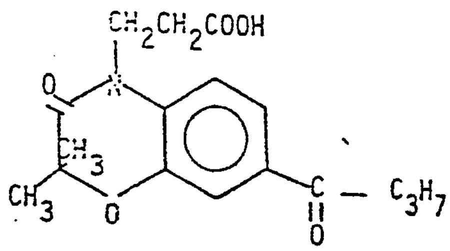

Mode opératoire :
Porter au reflux pendant 105 minutes, un mélange réactionnel composé de 0,02 mole de NM 14, de 10 $cm^3$ d'acide acétique, de 10 $cm^3$ d'acide sulfurique concentré et de 10 $cm^3$ d'eau.
Après refroidissement, ajouter 20 $cm^3$ d'eau.
Essorer et laver le précipité formé à l'eau. Sécher et recristalliser.
P.M. = 311,36 pour $C_{17}H_{21}NO_5$
P.F. = 146°C
Solvant de recristallisation : éthanol à 50°
Rendement : 77%

27/ (hydroxy-4 butyryl)-7 méthyl-4 benzoxazinone (MZ 48)

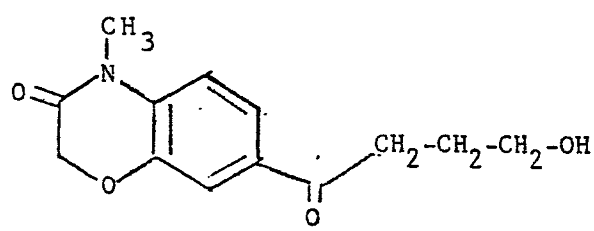

P.M. = 249,25 pour $C_{13}H_{15}NO_4$
P.F. = 130°C
Solvant de recristallisation : alcool absolu
Rendement : 75%

28/ (hydroxy-4 butyryl)7 trimethyl 2,2,4 benzoxazinone (MZ 150)

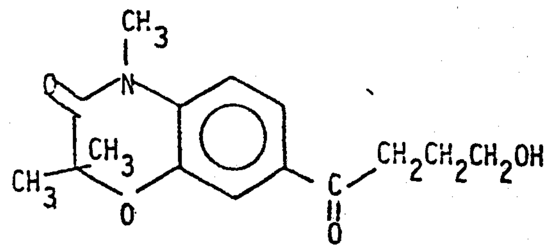

P.M. = 277,31 pour $C_{15}H_{19}NO_4$
P.F. = 83°C
Solvant de recristallisation : eau
Rendement : 60%

29/ (bromo-4 butyryl)-7 méthyl-4 benzoxazinone (MZ 49)

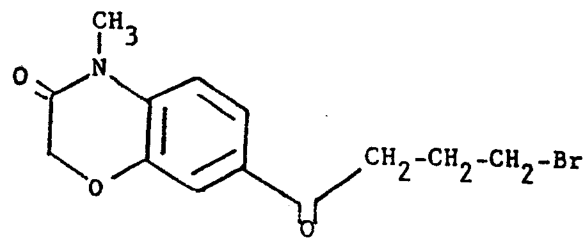

P.M. = 312,25 pour $C_{13}H_{14}NO_3Br$
P.F. = 122-124°C
Solvant de recristallisation : alcool absolu
Rendement : 80%
Il est préparé par action de l'acide bromhydrique gazeux sur le composé MZ 48 en opérant comme suit :
- Dissoudre le composé MZ 48 dans le benzène anhydre.
Tout en assurant une agitation magnétique modérée, faire barboter l'acide bromhydrique dans la solution en surveillant la prise de poids et abandonner le milieu réactionnel dans un bain d'huile à 40°C et sous agitation pendant deux heures. Filtrer et évaporer le solvant sous vide et recristalliser dans l'alcool absolu.

30/ (bromo-4 butyryl)7 triméthyl 2,2,4 benzoxazinone (MZ 151)

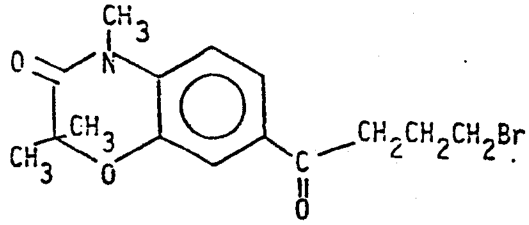

P.M. = 340,30 pour $C_{15}H_{18}NO_3Br$
P.F. = 111°C
Solvant de recristallisation : alcool absolu

Rendement : 80%

31/ [(diméthylamino)-4 butyryl]-7 méthyl-4 benzoxazinone (MZ 50)

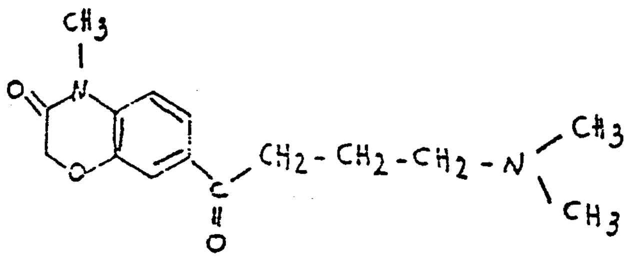

On peut procéder par condensation des amines :

- Dissoudre par chauffage la (bromo-4 butyryl)-7 méthyl-4-benzoxazinone dans le benzène anhydre. Ajouter sous agitation l'amine correspondante, puis une quantité équivalente de trièthylamine. Chauffer à reflux pendant quinze heures, puis essorer à chaud le précipité formé (sel de triethylamine).

Evaporer le filtrat sous vide et recristalliser le résidu.

P.M. = 276,32 pour $C_{15}H_{20}N_{2}O_{3}$

P.F. = 98-100°C

Solvant de recristallisation : hexane

Rendement : 50%

32/ [(diméthylamino)4 butyryl] triméthyl 2,2,4 benzoxazinone (MZ 154)

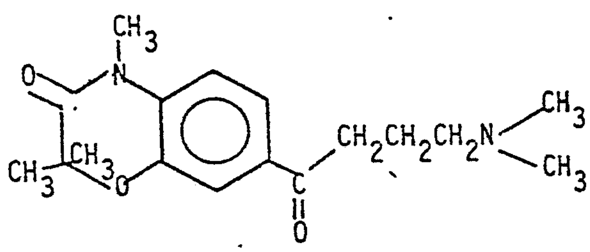

P.M. = 304,38 pour $C_{17}H_{24}N_{2}O_{3}$

P.F. = 67°

Solvant de recristallisation : hexane

Rendement : 50%

33/ [(phényl-4' piperazino)4 butyryl]7 triméthyl 2,2,4 benzoxazinone (MZ 152)

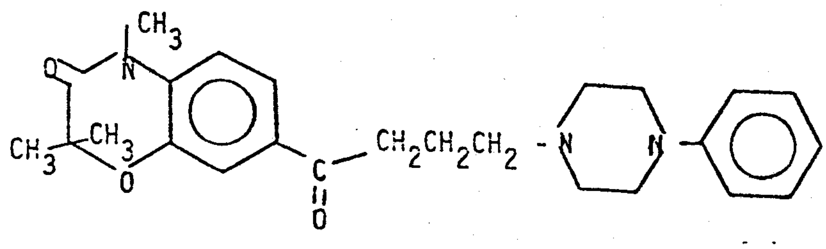

P.M. = 421,52 pour $C_{25}H_{31}N_3O_3$
P.F. = 131°C
Solvant de recristallisation : alcool absolu
Rendement : 65%

34/ [(p fluorophényl-4' piperazino)4 butyryl]7 triméthyl 2,2,4 benzoxazinone (MZ 153)

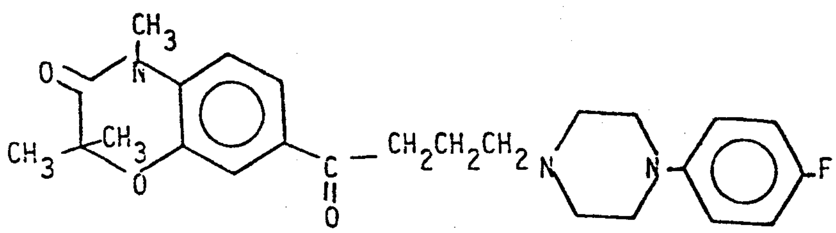

P.M. = 439,51 pour $C_{25}H_{30}FN_3O_3$
F = 82-84°C
Solvant de recristallisation : isopropanol
Rendement : 60%

35/ [(m Méthoxyphenyl-4' pipérazino)4 butyryl]7 triméthyl 2,2,4 benzoxazinone (MZ 155)

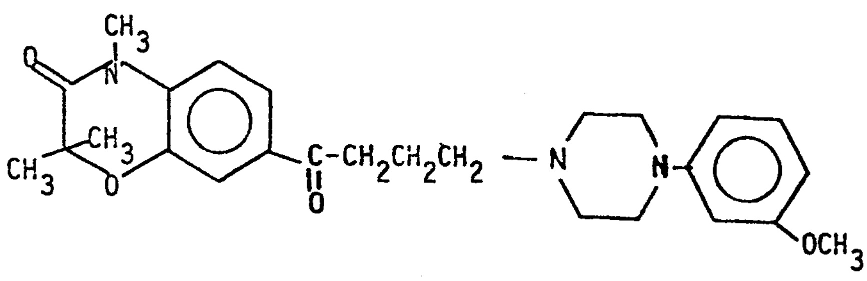

P.M. = 451,55 pour $C_{26}H_{33}N_3O_4$
P.F. - 118°C
Solvant de recristallisation : méthanol
Rendement = 55%

36/ Cyclopropoyl-7 méthyl-4 benzoxazinone (MZ 82)

On prépare d'abord l'acyl-5 amino-2 phénol MZ 83 de formule :

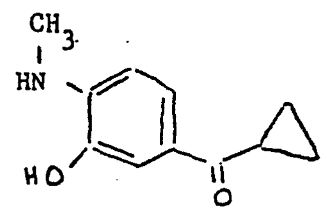

à partir de la (bromo-4 butyryl)-6 méthyl-3 benzoxazolinone en la traitant en milieu basique fort, plus particulièrement par une solution d'hydroxyde de sodium à 20%. On chauffe pendant une heure à 80°C, puis on refroidit cette solution et on la neutralise par une solution d'acide chlorhydrique dilué (pH 6,5-7). On a donc de cette façon simultanément effectué une ouverture du cycle benzoxazolinonique et une déshalohydrogénation en position alpha de son groupement cétonique. Les caractéristiques chimiques du produit MZ 83 sont les suivantes :

P.M. = 191,22 pour $C_{12}H_{13}NO_2$

P.F. = 170°C

Solvant de recristallisation : alcool dilué au demi.

A partir du produit MZ 83 on a ensuite formé le produit MZ 82 de formule :

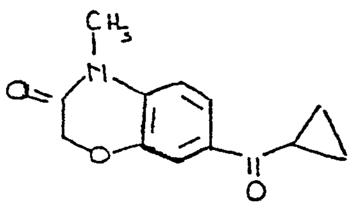

en mettant en oeuvre le protocole opératoire de l'exemple I

P.M. = 231,24 pour $C_{13}H_{13}NO_3$

P.F. = 110-112°C

Solvant de recristallisation : cyclohexane ou alcool à 95°

Rendement : 80%

37/ cyclopropyl-7 diméthyl 2,4 benzoxazinone (MZ 87)

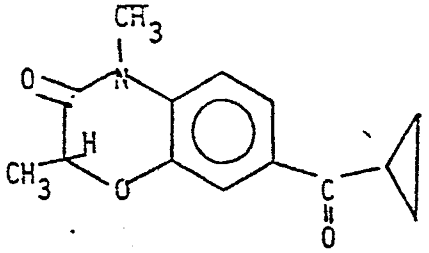

P.M. = 245,52 pour $C_{14}H_{15}NO_2$

P.F. = 87°C

Solvant de recristallisation : cyclohexane

Rendement : 80%

38/ cyclopropyl-7 triméthyl 2,2,4 benzoxazinone (MZ 88)

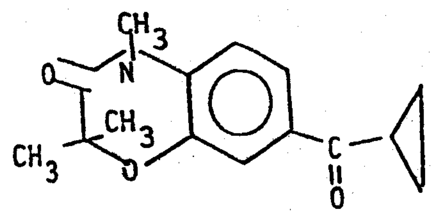

P.M. = 259,29 pour $C_{15}H_{17}NO_2$
P.F. = 51-53°C
Solvant de recristallisation : hexane
Rendement : 50%

39/ Thénoyl-7 méthyl-4 benzoxazinone (MZ 145)

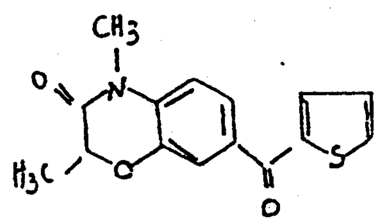

Il est préparé dans des conditions semblables à celles qui ont été envisagées dans les exemples précédents.
P.M. = 287,32 pour $C_{15}H_{13}NO_3S$
P.F. = 120-124°C
Solvant de recristallisation : alcool absolu ou isopropanol ou propanol
Rendement : 75%

EXEMPLE II : Préparation de "composés du groupe II".
Le mode opératoire appliqué aux matières premières suivantes, est d'application générale :
- butyryl-7-méthyl-4 benzoxazinone
- N'N'N'N'-tétraméthyl diamino méthane
-anhydride acétique.

Dans un ballon rodé de 100 cm3, mettre en suspension 0,02 mole de dérivé de la butyryl-7 méthyl-4 benzoxazinone dans 10 cm3 de N'N'N'N'-tétraméthyl-diaminométhane. Ajouter goutte à goutte et sous agitation magnétique 10 cm3 d'anhydride acétique, et chauffer le milieu réactionnel à 90°C pendant deux heures. Refroidir à la température ambiante et verser le milieu réactionnel dans 300 cm3 d'eau glacée. Essorer le précipité et laver à l'eau jusqu'à neutralité du filtrat. Sécher et recristalliser dans un solvant convenable.

1/ (Méthylène-2 butyryl)-7 méthyl-4 benzoxazinone (MZ 78)

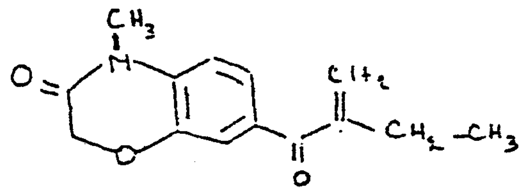

P.M. = 245,27 pour $C_{14}H_{15}NO_3$
P.F. = 76-78°C
Solvant de recristallisation : méthanol
Rendement : 80%

2/ (Méthylène-2 butyryl)-7 diméthyl-2,4 benzoxazinone (MZ 79)

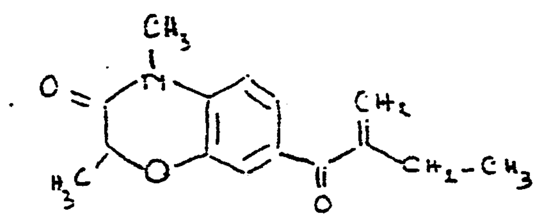

P.M. = 259,29 pour $C_{15}H_{17}NO_3$
P.F. = 51-52°C
Solvant de recristallisation : éther
Rendement : 80%

3/ (Méthylène-2 butyryl)-7 triméthyl-2,2,4 benzoxazinone (MZ 77)

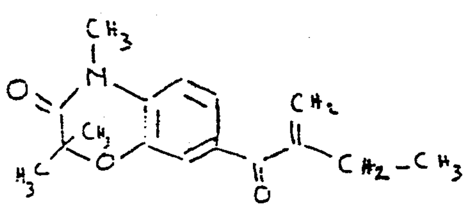

P.M. = 273,32 pour $C_{16}H_{19}NO_3$
P.F. = 66-68°C
Solvant de recristallisation : hexane ou alcool absolu
Rendement : 80%

4/ [(phenyl-4' pipérazino)4 méthylène-2 butyryl]7 triméthyl 2,2,4 benzoxazinone (MZ 158)

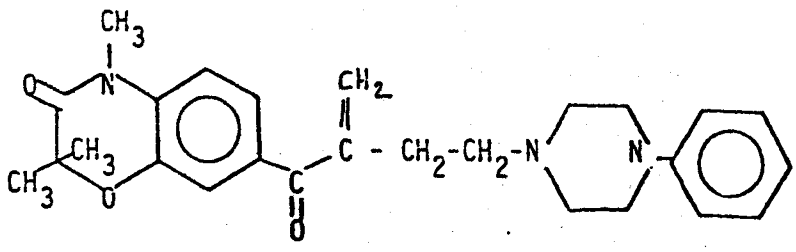

P.M. = 433,55 pour $C_{26}H_{31}N_3O_3$
P.F. = 82-84°C
Solvant de recristallisation : éthanol absolu
rendement : 50%

5/ [Methylène-2 acétoxy-3 propionyl]7 triméthyl 2,2,4 benzoxazinone (MZ 147)

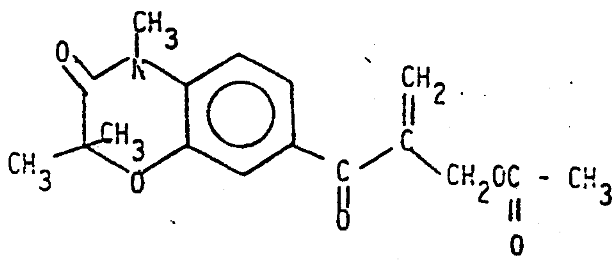

P.M. = 317,33 pour $C_{17}H_{19}NO_5$
P.F. = 110°C
Solvant de recristallisation : méthanol
Rendement : 55%

EXEMPLE III : Préparation de "composés du groupe III"

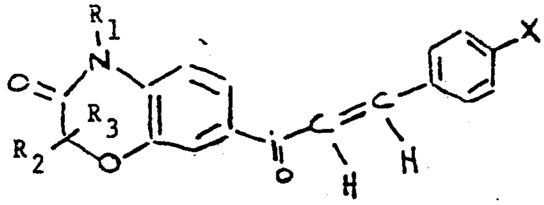

- Mode opératoire utilisé :

Dissoudre 0,03 mole de l'acyl-7 benzoxazinone dans 150 cm3 d'éthanol absolu saturé d'acide chlorydrique gazeux, puis ajouter lentement, et sous agitation magnétique, 0,035 mole de l'aldéhyde correspondant. Poursuivre l'agitation pendant deux heures. Le produit attendu précipite dans le milieu réactionnel. Essorer et laver le précipité par de l'alcool absolu, puis par de l'eau jusqu'à neutralité du filtrat. Sécher et recristalliser dans un solvant convenablement choisi.

1/ (Chloro-4 cinnamoyl)-7 méthyl-4 benzoxazinone)
(MZ 129)

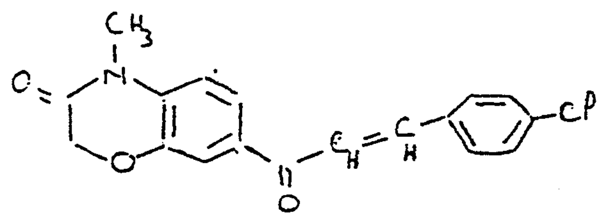

P.M. = 327,81 pour $C_8H_{14}ClNO_3$
P.F. = 209°
Solvant de recristallisation : acétone
Rendement : 70%

2/ (Chloro-4 cinnamoyl)7 diméthyl 2,4 benzoxazinone
(MZ 134)

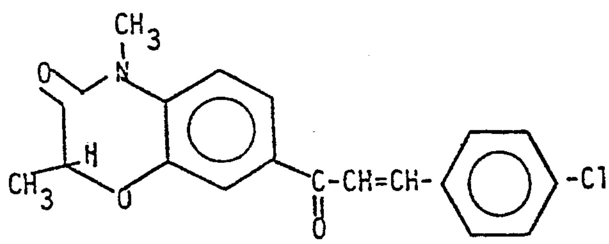

P.M. = 341,82 pour $C_{19}H_{16}ClNO_3$
P.F. = 125°C
Solvant de recristallisation : alcool absolu
Rendement : 70%

3/ (Chloro-4 cinnamoyl)7 triméthyl 2,2,4 benzoxazinone
(MZ 137)

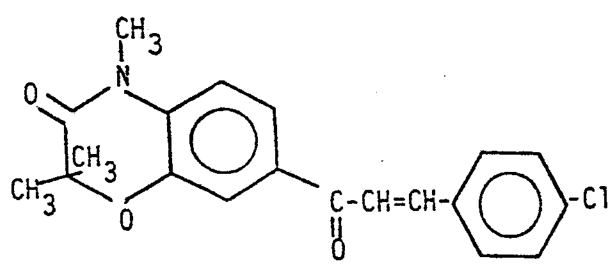

P.M. = 355,85 pour $C_{20}H_{18}ClNO_3$
P.F. = 166-167°C
Solvant de recristallisation : éthanol absolu
Rendement : 65%

4/ (Hydroxy-4 cinnamoyl)-7 méthyl-4 benzoxazinone) (MZ 130)

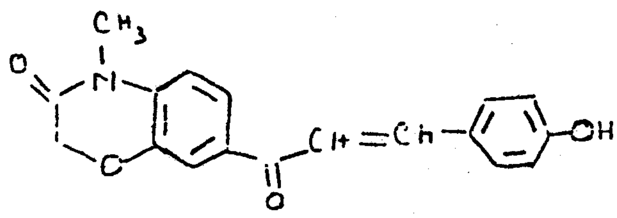

P.M. = 309,30 pour $C_{18}H_{15}NO_4$
P.F. = 222-225°C
Solvant de recristallisation : acétone
Rendement : 70%

5/ <u>(hydroxy-4 cinnamoyl)7 diméthyl 2,4 benzoxazinone</u>
(MZ 133)

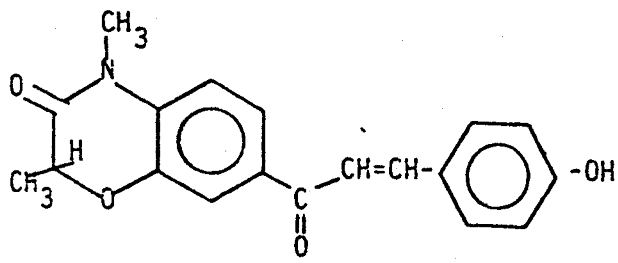

P.M. = 323,33 pour $C_{19}H_{17}NO_4$
P.F. = 226°C
Solvant de recristallisation : acétone
Rendement : 75%

6/ <u>(hydroxy-4 cinnamoyl)7 triméthyl 2,2,4 benzoxazinone</u>
(MZ 139)

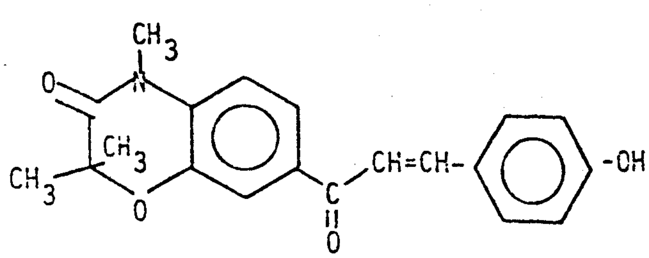

P.M. = 337,36 pour $C_{20}H_{19}NO_4$
P.F. = 228°
Solvant de recristallisation : acétone
Rendement : 60%

7/ Cinnamoyl-7 méthyl-4 benzoxazinone (MZ 131)

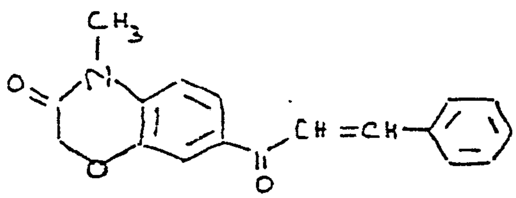

P.M. = 293,30 pour C18H15NO3
P.F. = 134-136°C
Solvant de recristallisation : alcool absolu
Rendement : 70%.

8/ Cinnamoyl-7 diméthyl 2,4 benzoxazinone (MZ 135)

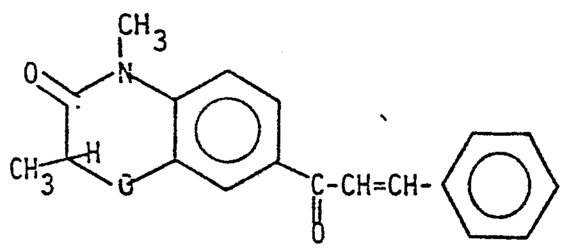

P.M. = 307,33 pour $C_{19}H_{17}NO_3$
P.F. = 135°C
Solvant de recristallisation : éthanol absolu
Rendement : 70%

9/ Cinnamoyl-7 triméthyl 2,2,4 benzoxazinone (MZ 138)

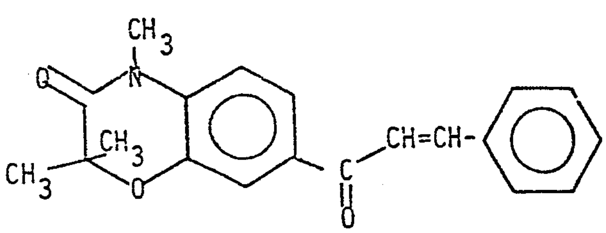

P.M. = 321,36 pour $C_{20}H_{19}NO_3$
P.F. = 136-138°C
Solvant de recristallisation : éthanol absolu
Rendement : 65%

EXEMPLE IV : Préparation des composés du groupe IV

Mode opératoire utilisé :

Des dérivés de cétones alpha-bêta éthyléniques ("composés du groupe II") sont utilisés comme matières premières. Dissoudre 0,03 mole de la cétone alpha-bêta éthylénique dans 30 ml d'acide sulfurique concentré

(98%) puis abandonner le milieu réactionnel sous agitation à température ambiante (18-25° C) durant quinze heures. Verser ce milieu dans 300 ml d'eau glacée. Essorer le précipité formé dans ce milieu, et laver à plusieurs reprises par de l'eau froide jusqu'à neutralité du filtrat. Sécher et recristalliser dans un solvant convenable.

1/ 4H-dihydro-2,3-dioxo-3,8-méthyl-4-cyclopenta (g)-benzoxazine (MZ 84)

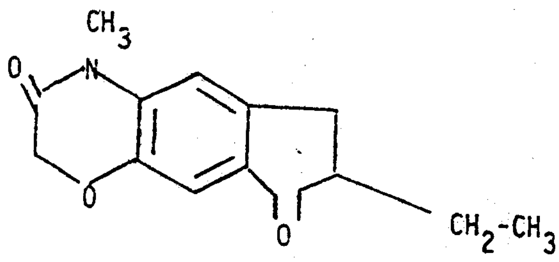

P.M. = 245,27
P.F. = 147°C
Solvant de recristallisation : acétone
Rendement : 90%

2/ 4H dihydro 2,3 dioxo 3,8 triméthyl 2,2,4 méthylène 7 cyclopenta (g) benzoxazine (MZ 148)

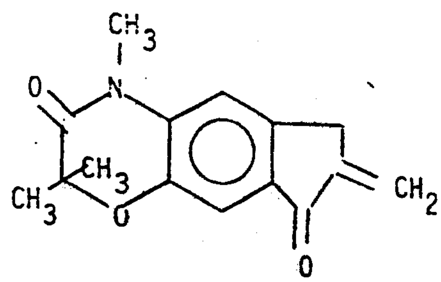

P.M. = 257,27 pour $C_{15}H_{15}NO_3$
P.F. = 184°
Solvant de recristallisation : acétone
Rendement : 65%

3/ <u>4H dihydro 2,3 dioxo 3,8 diméthyl 2,4 éthyl</u> 7 <u>cyclopenta (g) benzoxazine (MZ 89</u>)

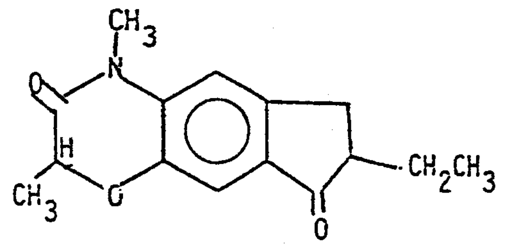

P.M. = 259,29 pour $C_{15}H_{17}NO_3$
P.F. = 120-122°C
Solvant de recristallisation : méthanol
Rendement : 75%

4/ <u>4H dihydro 2,3 dioxo 3,8 triméthyl 2,2,4 éthyl</u> 7 <u>cyclopenta (g) benzoxazine (MZ 90</u>)

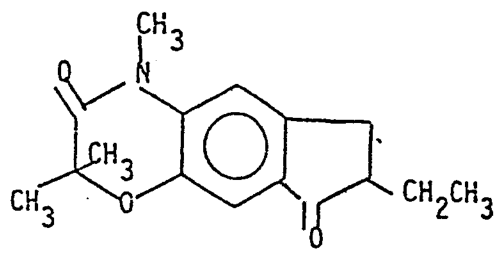

P.M. = 273,32 pour $C_{16}H_{19}NO_3$
P.F. = 110-112°C
Solvant de recristallisation : méthanol
Rendement : 80%

RESULTATS RELATIFS AUX PROPRIETES BIOLOGIQUES DES COMPOSES SELON L'INVENTION

L'étude pharmacologique des composés de l'invention a montré qu'ils possèdent d'intéressantes propriétés.

1/ TOXICITE :

La toxicité des composés a été déterminée par voie orale, chez le rat.

Tous les composés ont une $DL_{50}$ supérieure à 2000 mg/kg.

2/ ACTIVITE ANTIATHEROSCLEREUSE :

Des efforts considérables sont déployés depuis quelque temps pour trouver des substances pouvant s'opposer aux conséquences de l'hyperlipémie : hypercholestérolémie, déséquilibre du rapport apoprotéine $A_1$/B ou du rapport LDL Cholestérol/HDL CHolestérol (avec LDL: low density lipoproteins et HDL : high density lipoproteins) ces lipoprotéines étant séparées par gradients de densité). La répercussion la plus importante de l'hyperlipémie est l'athérosclérose. C'est la cause la plus fréquente des troubles coronariens, des artériopathies, des affections vasculaires cérabrales...

L'étude de FRAMINGHAM(Castelli W.P. et col., Circulation 1977, 55, 767.772.) a permis d'établir une corrélation entre des taux élevés de cholestérol total et une augmentation du risque d'attaque cardiaque ainsi que la corrélation entre taux élevés d'HDL cholestérol et diminution de ce même risque.

La prévention, l'arrêt ou le traitement de l'athérosclérose nécessite de traiter l'hyperlipémie.

De nombreux agents hypolipémiants sont maintenant disponibles : clofibrate, cholestyramine, gempibrozil, probucol...

Le développement de nouveaux produits dépourvus d'effets secondaires, capables de réduire le cholestérol total, les triglycérides, de rétablir les balances $ApoA_1$/Apo B, HDL cholestérol/LDL cholestérol et $TXA_2/PGI_2$, $TXA_2$ étant le thromboxane $A_2$ et $PGI_2$ étant la prostacycline, est souhaité par la communauté

médicale pour le traitement et la prévention de l'athérosclérose. Des formes orales sont nécessaires car les patients sont appelés à prendre ces médicaments pendant de nombreuses années.

Les produits de cette invention sont originaux et possèdent des propriétés pharmacologiques intéressantes.

Ils présentent une structure chimique originale et ont une activité supérieure aux autres hypolipémiants actuellement commercialisés. Ces nouveaux composés sont bien absorbés par le tractus gastro intestinal et sont dépourvus d'effets secondaires notamment une absence d'hépatomégalie et de prolifération de péroxysomes.

Ces produits ont été testés pour confirmer leur activité vis-à-vis des perturbations athéromateuses.

Les différents points ci-dessous ont été étudiés :

- Test au Triton, détergent tensioactif commercialisé sous la dénomination WR 1339 chez le rat.
- Activité des molécules sélectionnées par le test au Triton sur des souris rendues hypercholestérolémiques et des souris normales : étude des paramètres lipidiques, de la composition lipoprotéinique et apoprotéinique.
- Etude préliminaire sur le poids du foie et l'examen histologique du tissu hépatique.
- Activité antiagrégante plaquettaire selon les tests classiques (in vitro, sur sang humain).

3/ ACTIVITE NORMOLIPEMIANTE :

3.1. L'activité normolipémiante des composés a été mise en évidence par la méthode classique du test mettant en jeu le détergent comme dans la désignation commerciale TRITON. Cette méthode est décrite dans les articles suivants : Kellner A. et Coll. J. of Experimental Med. 1951, vol 93, p. 373-385 et dans Schurr P.E. et Coll. Lipids 1952, vol 7, n° 1, 68-74.

Les résultats sont résumés dans le tableau suivant.

Tous les composés ont été administrés par voie orale au rat, à raison de 300 mg/kg. A titre de comparaison, on fournit les résultats obtenus avec le fénofibrate à la même dose.

Les résultats obtenus sont à l'avantage des composés selon l'invention, surtout si l'on tient compte de la totale absence de toxicité qui les caractérise. On peut noter aussi la meilleure activité des dérivés substitués en 7 par rapport à leur homologue substitué en 6 ainsi que celle des dérivés acylés sur le noyau aromatique par rapport à leurs homologues réduits.

## VARIATION DES % D'ACTIVITE PAR RAPPORT AU TEMOIN TRITON

| | Cholestérol total | Cholestérol HDL | Triglycérides | Apo B |
|---|---|---|---|---|
| MZ 77 | 50,1 | 29 | 85 | 15,3 |
| MZ 78 | 54,6 | 72,4 | 89,7 | 34,2 |
| MZ 79 | 56,9 | 72,4 | 89,3 | 35 |
| MZ 86 | 46,3 | 69,3 | 67,5 | 43,8 |
| Homologue du MZ 86 substitué en 6 | 28,0 | 30,0 | 15,0 | ND |
| MZ 95 | 33,0 | 15,6 | 48,0 | 26,8 |
| MZ 114 | 52,5 | 25,9 | 74,2 | ND |
| MZ 115 | 48,2 | 46,6 | 78,3 | ND |
| MZ 116 | 43,1 | 20,4 | 68,9 | ND |
| MZ 117 | 45,1 | 43,9 | 72,0 | ND |
| MZ 118 | 54,6 | 18,8 | 77,3 | ND |
| MZ 118 réduit | 15,0 | 29,0 | 25,0 | ND |
| MZ 119 | 50,1 | 43,3 | 73,9 | ND |
| MZ 120 | 19,2 | ND | 19,7 | 14,3 |
| MZ 121 | 26,5 | 7,9 | 45,3 | 26,2 |
| MZ 122 | 24,2 | 0 | 37,3 | 23,8 |
| MZ 123 | 34,8 | 16,7 | 50,1 | 27,9 |
| MZ 124 | 36,5 | 19,2 | 61,1 | 29,2 |
| MZ 125 | 26,8 | 10,3 | 47,0 | 26,7 |
| MZ 126 | 19,5 | 13,2 | 20,5 | 21 |
| MZ 129 | 63,3 | 29,8 | 81,8 | 32 |
| MZ 130 | 55,7 | 44,8 | 75,5 | 35 |
| MZ 131 | 62,9 | 23 | 82,6 | 32 |
| MZ 132 | 22,2 | 21,5 | 5,8 | 17 |
| MZ 136 | 31,0 | 42,0 | 29,5 | ND |
| Fénofibrate | 36,5 | 48,3 | 64,5 | 19,5 |

3.2. L'activité normolipémiante a aussi été mise en évidence chez les souris $OF_1$ normales ou rendues hypercholestérolémiques. Les produits testés sont administrés per os chaque jour pendant 15 jours à la dose

de 50 mg/kd. L'évolution pondérale est observée en cours de traitement, un bilan lipidique est effectué en fin d'expérimentation par rapport à un groupe témoin.

Les résultats sont résumés dans le tableau suivant. Tous les composés ont été administrés par voie orale à raison de 50 mg/kg.

On sait que les HDL constituent la "fraction épuratrice" du cholestérol. Les HDL transportent le cholestérol pour le transformer au niveau du foie en sels biliaires qui seront éliminés par la bile.

| TEMOIN | Poids Foie/ Poids corporel(%) | CT (1) % | $HDL_C$/CT % (2) | TG(3) % | $HDL_C$ (4) % | Phospho Lipides |
|---|---|---|---|---|---|---|
| FENOFI-BRATE | + 55% | -30% | +30% | +24% | -11% | +18% |
| MZ 86 | + 2% | -38% | +44% | -12% | +18% | 0% |
| MZ 95 | + 6% | -21% | +25% | - 5% | 0 | + 5% |
| MZ 115 | + 9% | -32% | +17% | +18% | - 5% | - 9% |
| MZ 119 | + 12% | -30% | +21% | +21% | - 6% | +15% |
| MZ 125 | + 17% | -25% | +18% | +19% | + 1% | +15% |
| MZ 134 | + 5% | - 5% | +10% | - 9% | + 5% | +11% |
| MZ 153 | + 4% | -10% | +45% | +18% | +25% | +19% |
| NM 7 | - 10% | + 2% | +14% | + 5% | + 5% | + 5% |
| NM 8 | - 15% | + 8% | +11% | +23% | +10% | + 8% |
| NM 11 | 0% | -37% | +43% | - 8% | + 7% | +16% |

(1) CT = cholestérol total

(2) HDL (c)/CT = rapport entre le cholestérol contenu dans les HDL et le cholestérol total

(3) Triglycérides

(4) Fraction de cholestérol contenue dans les HDL

3.3 Activité inhibitrice de l'agrégation plaquettaire.

L'effet inhibiteur de diverses B aminocétones sur l'agrégation plaquettaire a déjà été décrit. (CAZIN et col. Acta Therapeutica 1980, 6, 205-211). Les études pharmacologiques réalisées sur certaines molécules de la présente invention ont confirmé que cette activité inhibitrice se traduit par une activité antithrombotique et anti-agrégante plaquettaire. Cette activité est très intéressante surtout si elle est associée à une activité hypolipémiante, elle permet une nouvelle approche de la thérapeutique antiathéroscléreuse.

Inhibition de l'agrégation plaquettaire in vitro : selon le protocole décrit par ASHIDA et col. Thrombosis Haemostasis 1979, 40, 542.

ACTIVITE INHIBITRICE DE L'AGREGATION PLAQUETTAIRE INDUITE PAR L'ARACHIDONATE DE SODIUM ET LE COLLAGENE.

| Référence produit | Arachidonate $DE_{50}$(mg/kg) | Collagène $DE_{50}$ (mg/kg) |
|---|---|---|
| Aspirine | 37 | 59,5 |
| NM 7 | 1,92 | 2,72 |
| NM 8 | 11,61 | 15,00 |
| MZ 130 | 0,15 | 0,19 |
| MZ 154 | 0,26 | 0,92 |
| MZ 133 | 6,10 | 11,00 |
| MZ 85 | 21,00 | 22,00 |

4/ AUTRES ACTIVITES PHARMACOLOGIQUES :

Lors des tests pharmacologiques préliminaires, un certain nombre d'autres activités ont été mises en évidence chez certains produits notamment des propriétés analgésiques, sédatives, antiinflammatoires, antimicrobiennes, antifongiques, cardiovasculaires notamment sur les récepteurs dopaminergiques, et sur le système nerveux central.

L'invention concerne plus particulièrement les compositions pharmaceutiques mettant en jeu les composés selon l'invention en association avec un véhicule pharmaceutiquement acceptable.

De préférence, les compositions selon l'invention se présentent sous forme administrable, de préférence orale, et notamment sous forme solide.

Elles peuvent être présentées sous toutes formes appropriées à ce mode d'administration, par exemple sous forme de comprimés, tablettes, etc.

Exemple de formulation galénique :
Butyryl-7 trimethyl-2,2,4 benzoxazinone : 100 mg
Stéarate de magnésium : 1 mg
Talc : 5 mg
Amidon de maïs : 20 mg
Poids du contenu de la gélule : 126 mg

Les composés selon l'invention sont utiles pour le traitement d'affections cardio-vasculaires impliquant notamment les lipides ou glycérides.

Par ailleurs, les compositions selon l'invention se caractérisent par une activité analgésique, anti-inflammatoire, sédative, antibactérienne, antifongique, cardiovasculaire et sur le système nerveux central.

La totale absence de toxicité les font bénéficier d'un index thérapeutique particulièrement favorable.

Les compositions sont avantageusement préparées de façon à permettre l'administration de doses unitaires 1 à 500 mg, s'agissant plus particulièrement de la voie orale. Des intervalles de doses quotidiennes utiles chez l'adulte peuvent s'établir entre 5 et 1000 mg par jour.

Il va de soi qu'entrent dans le champ de l'invention revendiquée tous autres dérivés présentant les structures de base principales ou préférées qui ont été envisagées dans le cadre de cette description. De ce point de vue, font partie de l'invention revendiquée les molécules qui se distingueraient de celles qui sont plus particuliérement revendiquées, seulement par la présence de substituants non expressément identifiés dans les revendications, mais qui ne seraient par de nature à sensiblement modifier les propriétés biologiques des dérivés en cause.

L'invention concerne aussi l'utilisation des composés sus-indiqués pour la fabrication de médicaments destinés à être utilisés pour des traitements d'infections cardio-vasculaires, impliquant notamment les lipides ou les glucides, ou destinés à être utilisés en tant qu'analgésiques anti-inflammatoires ou fongicides.

**Revendications**

1/ Dérivés d'acyl-7 benzoxazinones de formule générale :

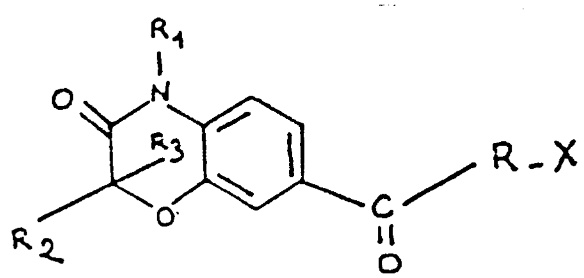

dans laquelle :

- $R_1$ est soit de l'hydrogène, soit un groupe hydrocarboné comprenant de 1 à 6 atomes des carbone, de préférence alkyle, alkaryle ou aralkyle, le cas échéant porteurs de substituants, notamment du type de ceux envisagés ci-après à propos du groupe X, soit un groupe hétérocyclique, notamment thiényle, furyle, pyridinyle ou pyridyle.
- $R_2$ et $R_3$ sont chacun, et ce de façon indépendante l'un de l'autre, de l'hydrogène ou un groupe alcoyle comprenant de 1 à 6 atomes de carbone.
- R est un groupe hydrocarboné formé d'éléments cycliques ou aliphatiques, ou des deux à la fois, comprenant de 1 à 15 atomes de carbone, ou l'un des susdits groupes hétérocycliques,
- X est de l'hydrogène ou un groupe carboxyle, halogène, hydroxyle, nitro, aminé, lui-même substitué ou non par un ou deux groupes alcoyle comprenant de 1 à 4 atomes de carbone ou un groupe aminé hétérocylique, alcoxyle comprenant de 1 à 4 atomes de carbone, X pouvant encore définir une liaison

chimique de cyclisation formée entre R et l'atome de carbone en position 6 du cycle benzénique de la partie benzoxazinone de la molécule, ainsi que, s'il y a lieu, les sels ou esters correspondants et les isomères optiques lorsque $R_2$ et $R_3$ sont différents, et quand X est l'hydrogène ou le brome, d'une part, et R est $CH_2$, d'autre part, $R_1$, $R_2$ et $R_3$ ne peuvent être simultanément tous de l'hydrogène.

2/ Dérivés selon la revendication 1, caractérisés en ce que $R_1$, $R_2$ et $R_3$ sont, indépendamment les uns des autres, des hydrogènes ou des groupes méthyle.

3/ Dérivés selon la revendication 1 ou la revendication 2, caractérisés en ce que R est un groupe aryle notamment phényle, arylalkyl ou hétérocyclique, alkaryle notamment benzyle, alkyle y compris cyclopropyle, comprenant de 1 à 6 atomes de carbone ou l'un des susdits groupes hétérocycliques, X étant de l'hydrogène ou un halogène, notamment le chlore ou le brome, un hydroxyle ou un groupe aminé, substitué ou non, ou encore l'un des susdits groupes hétérocycliques.

4/ Dérivés selon la revendication 1 ou la revendication 2, caractérisés en ce que le groupe R est une chaîne aliphatique dont le premier carbone, en alpha du groupe carbonyle, est porteur d'un groupe de remification éthylénique.

5/ Dérivés selon l'une quelconque des revendications 1 à 3, caractérisés en ce que X est un groupe aminé hétérocyclique notamment la pipérazine substitué ou non.

6/ Dérivés selon l'une quelconque des revendications 1 à 5 caractérisés en ce que le groupe R lié à la fonction cétone de la chaîne latérale porte au moins 3 atomes de carbone.

7/ Dérivés selon l'une quelconque des revendications 1 à 5 caractérisés en ce que le groupe R lié à la fonction cétone de la chaîne latérale porte au moins 4 atomes de carbone.

8/ Dérivés selon la revendication 1, caractérisés en ce que le groupe CO-R est un groupe méthylène-2-butyryle.

9/ Dérivés selon la revendication 1 ou la revendication 2, caractérisés en ce que R est un groupe -C=C-W-X, dans lequel W est un groupe alkyle, arylalkyle, alkaryle ou aryle.

10/ Dérivés selon la revendication 9, caractérisés en ce que R est un groupe $-C=C-C_6H_5-X$, X étant de l'hydrogène, un halogène, un groupe amino, hydroxyle, méthoxyle ou nitro.

11/ Dérivés selon la revendication 1 ou la revendication 2, caractérisés en ce que R est une chaîne aliphatique comprenant 2 ou 3 atomes de carbone, éventuellement ramifiée par un groupe de préférence alcoyle comprenant de 1 à 4 atomes de carbone, et formant une liaison de cyclisation de cette chaîne avec l'atome de carbone en position 6 sur le cycle benzénique du groupe benzoxazinone.

12/ Composition pharmaceutique contenant un dérivé selon l'une quelconque des revendications 1 à 11, en association avec un véhicule pharmaceutiquement acceptable.

13/ Composition pharmaceutique selon la revendication 12, caractérisée en ce qu'elle se présente sous forme de composition solide destinée à l'administration orale, cette composition se caractérisant par des doses unitaires de 1 à 500 mg.

14/ Composition pharmaceutique pour le traitement des affections athéromateuses contenant un dérivé selon l'une des revendications 1 à 11, en association avec un véhicule pharmaceutiquement acceptable.

15/ Utilisation de la substance selon l'une des revendications 1 à 11 pour la fabrication d'un médicament destiné au traitement des affections athéromateuses.

Office européen des brevets

# RAPPORT DE RECHERCHE EUROPEENNE

Numero de la demande

EP 86 40 2355

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| Y | US-A-4 358 455 (J.G. ATKINSON et al.)<br>* Colonnes 13,14, exemples 1A-B *<br>--- | 1,2 | C 07 D 265/36<br>C 07 D 265/34<br>C 07 D 413/06<br>A 61 K 31/535 |
| Y | THE JOURNAL OF ORGANIC CHEMISTRY, vol. 48, no. 25, 16 décembre 1983, pages 5140-5143, American Chemical Society; W.R. BAKER: "Alkoxide-Accelerated Smiles Rearrangements. Synthesis of N-(2-Hydroxyethyl)anilines from N-(2-Hydroxyethyl)(aryloxy)acetamides"<br>* Page 5141, formule 8 *<br>--- | 1-3 | |
| A | US-A-3 770 734 (M. PESSON et al.)<br>* Résumé; revendications *<br>--- | 1,12 | **DOMAINES TECHNIQUES RECHERCHES (Int Cl 4)** |
| A | DE-A-2 509 155 (HOECHST AG)<br>* Revendications *<br>--- | 1,9 | C 07 D 265/00<br>C 07 D 413/00<br>A 61 K 31/00 |
| A | FR-A-1 560 628 (LABORATOIRE R. BELLON)<br>* Résumé *<br>----- | 1 | |

Le present rapport de recherche a été établi pour toutes les revendications

| Lieu de la recherche | Date d'achèvement de la recherche | Examinateur |
|---|---|---|
| LA HAYE | 19-01-1987 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82